(19) Europäisches Patentamt
European Patent Office
Office européen des brevets

(11) Publication number: **0 018 203**
**B1**

(12) **EUROPEAN PATENT SPECIFICATION**

(45) Date of publication of patent specification: **02.03.83**

(21) Application number: **80301225.1**

(22) Date of filing: **14.04.80**

(51) Int. Cl.$^3$: **C 07 D 498/04,**
A 61 K 31/42,
A 61 K 31/43,
A 61 K 31/545
//C07D405/12, (C07D498/04, 263/00, 205/00), (A61K31/43, 31/42), (A61K31/545, 31/42)

(54) **Clavulanic acid derivatives, their preparation and use in pharmaceutical compositions.**

(30) Priority: **21.04.79 GB 7913949**

(43) Date of publication of application:
**29.10.80 Bulletin 80/22**

(45) Publication of the grant of the patent:
**02.03.83 Bulletin 83/9**

(84) Designated Contracting States:
**CH DE FR GB IT LI NL**

(56) References cited:
**DE - A - 2 633 561**
**DE - A - 2 702 954**
**DE - A - 2 708 330**

(73) Proprietor: **BEECHAM GROUP PLC**
**Beecham House Great West Road**
**Brentford Middlesex (GB)**

(72) Inventor: **Davies, John Sydney**
**17, Beaufort Road**
**Reigate Surrey (GB)**
Inventor: **Hunter, Pamela Ann**
**Caniper Cottage Mill Lane**
**Lower Beeding Horsham, Sussex (GB)**

(74) Representative: **Hesketh, Alan, Dr. et al**
**European Patent Attorney Beecham Pharmaceuticals Great Burgh Yew Tree Bottom Road**
**Epsom, Surrey, KT18 5XQ (GB)**

**EP 0 018 203 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Clavulanic acid derivatives their preparation and use in pharmaceutical compositions

This invention relates to novel antibiotics and β-lactamase inhibitors and to processes for their production.

Belgian Patent Specification Nos. 850779 and 851821 disclose that thioethers derived from clavulanic acid and oxidation products of such thioethers possess β-lactamase inhibitory properties. It has now been found that one thioether and its oxidation products, and pharmaceutically acceptable salts and esters thereof are particularly effective as β-lactamase inhibitors for the protection of penicillins and cephalosporins from degradation in animals infected by β-lactamase producing bacteria.

The present invention provides the compounds of the formula (I):

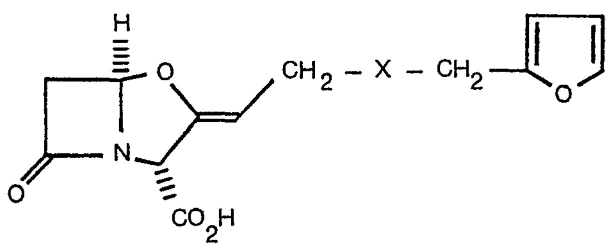

(I)

and salts and cleavable esters thereof wherein X is a sulphur atom or a SO or $SO_2$ group.

Suitably X in the compound of the formula (I) is a sulphur atom. More suitably X in the compound of the formula (I) is SO. Preferably X in the compound of the formula (I) is $SO_2$.

The compounds of the formula (I) are suitably provided in the form of the free-acid.

The compounds of the formula (I) are aptly in the form of a salt. Although such salts are normally those which are pharmaceutically acceptable, salts with pharmaceutically unacceptable ions are also useful as they may serve as intermediates in the preparation of pharmaceutically acceptable salts by ion-exchange or in the preparation of cleavable esters.

Examples of pharmaceutically acceptable salts of the compounds of the formula (I) include the alkali metal salts such as sodium or potassium, alkaline earth metal salts such as calcium or magnesium, and ammonium or substituted ammonium salts for example those with lower alkylamines such as triethylamine, hydroxy-lower alkylamines such as 2-hydroxyethylamine, bis-(2-hydroxyethyl)-amine, tris-(hydroxymethyl)amine or tris-(2-hydroxyethyl)-amine, cycloalkylamines such as bicyclohexylamine, or with procaine, dibenzylamine, N,N-dibenzylethylenediamine, 1-ephenamine, N-ethylpiperidine, N-benzyl-β-phenethylamine, dehydroabietylamine or N,N'-bis-dehydroabietylethylenediamine.

The lithium and t-butyl-ammonium salts are useful as intermediates, for example as salts which are easily prepared and stored in solid form and which later may be converted into a pharmaceutically acceptable salt as previously described by ion-exchange.

Thus suitable salts of this invention include the lithium, sodium, potassium, calcium, magnesium, ammonium and t-butylammonium salts. The sodium, potassium, calcium and magnesium salts are favoured pharmaceutically acceptable salts.

Highly favoured compounds of this invention includes salts of the compound of the formula (I) wherein X is a SO or $SO_2$ group. Preferred compounds of this invention include the pharmaceutically acceptable salts of the compound of the formula (I) wherein X is a $SO_2$ group.

The salts of the compounds of the formula (I) are envisaged primarily as pharmaceutical agents although they may also be employed as intermediates, for example in the preparation of other salts or in the preparation of the parent acid or in the preparation of esters.

The compounds of the formula (I) may be provided in the form of cleavable esters. Suitable cleavable esters include those which serve as chemical intermediates by way of being cleavable by chemical means such as hydrogenolysis or mild base hydrolysis. Other suitable cleavable esters are those hydrolysable in-vivo to a compound of the formula (I) or its salt.

Suitable esters of the compound of the formula (I) include those of the sub-formulae a) and b):

$$—CO_2A^1 \qquad a)$$

$$—CO_2CHA^2A^3 \qquad b)$$

wherein $A^1$ is an alkyl group of 1—6 carbon atoms optionally substituted by an alkoxyl or acyloxy group of 1—7 carbon atoms; $A^2$ is an alkenyl group of up to 5 carbon atoms or is a phenyl group optionally substituted by a fluorine, chlorine or bromine atom, or a nitro or alkyl or alkoxyl group of up to 4 carbon atoms; and $A^3$ is a hydrogen atom, an alkyl group of up to 4 carbon atoms or a phenyl group optionally

substituted by a fluorine, chlorine or bromine atom, or a nitro or alkyl or alkoxyl group of up to 4 carbon atoms.

Certain favoured groups $A^1$ include the methyl, ethyl, propyl, methoxymethyl, methoxyethyl, acetoxymethyl, acetoxyethyl, phthalidyl, ethoxycarbonyloxymethyl, $\alpha$-ethoxycarbonyloxyethyl and pivaloyloxymethyl groups.

Certain favoured groups $A^2$ include the phenyl, methoxyphenyl and nitrophenyl groups. A particularly favoured moiety $A^3$ is the hydrogen atom.

Of these esters those favoured as in-vivo hydrolysable esters are the acetoxymethyl, acetoxyethyl, phthalidyl, ethoxycarbonyloxymethyl, $\alpha$-ethoxycarbonyloxyethyl and pivaloyloxymethyl esters.

The in-vivo hydrolysable nature of the ester may be confirmed by administration to an animal such as a mouse or rat and determination of the presence of a compound of the formula (I) or a salt thereof in the blood or urine of the animal. Alternatively hydrolysis in human blood or serum may be determined.

The present invention also provides a pharmaceutical composition which comprises a compound of the formula (I) or a pharmaceutically acceptable salt or in-vivo hydrolysable ester thereof and a pharmaceutically acceptable carrier.

Most suitably the composition of this invention will contain a pharmaceutically acceptable salt of a compound of the formula (I). Favourably said salt is of the compound of the formula (I) wherein X is SO and preferably said salt is of the compound of the formula (I) wherein X is $SO_2$.

The compositions of this invention will generally contain from 25 mg to 500 mg of the compound of the invention but more usually from 50 mg to 250 mg of said compound, for example 75, 100, 125, 150, 175, 200 or 225 mg.

The compositions may be administered orally or by injection from 1 to 6 times a day and more usually from 2 to 4 times a day. The usual daily dose for a 70 kg adult human will be in the range 80 mg to 800 mgs and more usually from 125 mg to 500 mg.

Since the compounds of this invention have a degree of antibacterial activity in their own right they may be used to treat infections due to strains of Staphylococcus aureus, Escherichia coli, Klebsiella aerogenes, and Proteus spp.; for example they may be used to treat urinary tract infections due to such organisms or mastitis in cattle due to such organisms.

However as has been previously indicated it is believed that the greatest usefulness of the compounds of the formula (I) and their pharmaceutically acceptable salts and in-vivo hydrolysable esters lies in their ability to inactivate bacterial $\beta$-lactamase and so enhance the in-vivo effectiveness of penicillins or cephalosporins. It is possible to administer the above described compositions to a mammal which is concurrently or consecutively also treated with a penicillin or cephalosporin. In this form of therapy the penicillin or cephalosporin is used in its conventional dosage. However for greater convenience and better control it is preferred to include the penicillin or cephalosporin in the same unit dose composition as the compound of the formula (I) or its pharmaceutically acceptable salt or in-vivo hydrolysable ester.

Accordingly in a further aspect this invention provides a pharmaceutical composition which comprises a penicillin or cephalosporin, a compound of the formula (I) or a pharmaceutically acceptable salt or in-vivo hydrolysable ester thereof and a pharmaceutically acceptable carrier.

When used herein the term "penicillin" extends to the penicillin itself and to its pharmaceutically acceptable salts and pro-drugs. Similarly when used herein the term "cephalosporin" extends to the cephalosporin itself and to its pharmaceutically acceptable salts and pro-drugs.

The synergistic composition may be adapted for oral or parenteral administration except of course if the penicillin or cephalosporin included is not one orally administrable, when the synergistic composition will be adapted for parenteral administration.

British Patent Specification Nos. 1508977 and 1508978 disclose suitable forms of synergistic composition. By replacing the clavulanic acid or its salt or ester of said specifications by the compound of the formula (I) or its pharmaceutically acceptable salt or in-vivo hydrolysable ester, the desired composition of this invention results.

Particularly favoured orally administrable synergistic compositions of this invention are those which comprise amoxycillin trihydrate equivalent to 100 mg to 1000 mg of amoxycillin and the compound of the formula (I) wherein X is $SO_2$ or a pharmaceutically acceptable salt thereof equivalent to 50 mg to 500 mg of the compound of the formula (I) wherein X is $SO_2$.

The present invention also provides a process for the preparation of a compound of the formula (I) as hereinbefore defined or a salt or cleavable ester thereof which process comprises the reaction of a cleavable ester of a compound of the formula (II):

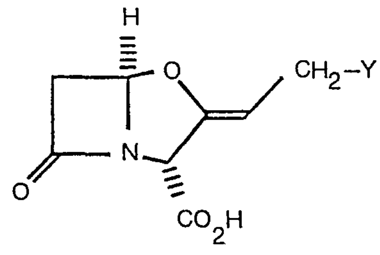

(II)

wherein Y is a displaceable atom or group; with furfurylmercaptan to yield the cleavable ester of the compound of the formula (I) wherein X is a sulphur atom, and thereafter optionally performing one or more of the following steps:

a) oxidising the sulphur atom to a SO or $SO_2$ group,

b) cleaving the cleavable ester to yield the compound of the formula (I) or its salt,

c) re-esterifying the compound of the formula (I) or its salt to form a different cleavable ester.

Suitable moieties Y include conventional displaceable groups such as sulphonate or carboxylate esters such as $O.SO_2.R^1$, $O.CO.R^1$ or $O.CO.OR^1$ where $R^1$ is an inert organic group such as a lower alkyl group (such as methyl, ethyl, propyl or butyl group) optionally substituted by one or more halogen atoms, a phenyl, benzyl, methoxyphenyl, methylphenyl, halophenyl or nitrophenyl group. Such displaceable groups may be displaced by furfurylmercaptan in the presence of a non-nucleophilic base, preferably 0.9—1.1 equivalents of such a base, and most preferably one equivalent. Suitably the non-nucleophilic base is triethylamine.

Other suitable moieties Y are halogen atoms, for example a chlorine or bromine atom, which may be displaced by furfurylmercaptan in the presence of a non-nucleophilic base and/or silver oxide and/or soluble silver salt preferably using 0.9—1.1 equivalents of such a base, and most preferably one equivalent. Suitably the non-nucleophilic base is triethylamine.

A further suitable moiety Y is the hydroxyl group which may be displaced by furfurylmercaptan in the presence of an acid catalyst. Suitably the acid catalyst is a Lewis acid catalyst such as boron trifluoride or its chemical equivalent (such as boron trifluoride etherate).

The thioetherification may be carried out at a non-extreme depressed or ambient temperature such as −60°C to +20°C, preferably from −30°C to 0°C.

When the thioetherification reaction comprises inter alia a non-nucleophilic base for example triethylamine, the reaction solvent is most suitably dimethylformamide. When the thioetherification reaction comprises an acid catalyst for example boron trifluoride the reaction medium is suitably a non-hydroxylic solvent such as a hydrocarbon, halohydrocarbon or ester solvent, for example benzene, toluene, dichloromethane, ethyl acetate, chloroform, or mixtures thereof.

Preferred values of Y when a non-nucleophilic base is used are a chlorine or bromine atom, or a dichloroacetoxy, mesyloxy, tosyloxy or phenylsulphonyloxy group. Particularly preferred is when Y is a dichloroacetoxy group.

A preferred value of Y when an acidic catalyst is used is when Y is a hydroxyl group.

Cleaving the cleavable ester of the compound of the formula (I) to form the parent acid or its salt may be achieved by hydrogenolysis, dissolving metal reduction or hydrolysis. Thus for example an ester such as a methyl, ethyl, methoxymethyl, ethoxymethyl or acetoxymethyl ester may be subjected to mild base hydrolysis to yield a salt of a compound of the formula (I). Suitably these esters may be hydrolysed by maintaining the pH of the medium at 7.5 to 9 until the hydrolysis is effected. Most suitably a readily hydrolysable ester such as the methoxymethyl ester is employed in this process. The pH may be maintained in the desired range in a pH-stat by the addition of a solution of suspension of a base such as LiOH, NaOH, KOH, $Ca(OH)_2$, $Mg(OH)_2$, $NaHCO_3$, $Na_2CO_3$ or $MgCO_3$ at a rate that prevents the accumulation of excess base which would cause the pH to increase unacceptably.

Dissolving metal reduction may be carried out using iron/ammonium chloride systems; but is not favoured for sulphoxides.

Suitable methods of hydrogenolysis of esters of the compounds of formula (I) include hydrogenation in the presence of a transition metal catalyst. Suitable hydrogenolysable esters of the compound of the formula (I) include those where the ester moiety is of the sub-formula $CO_2CHA^2A^3$ as hereinbefore defined and of these the benzyl and p-methoxybenzyl esters are particularly suitable. The p-nitrobenzyl ester is a preferred ester.

The pressure of hydrogen used in the reaction may be low, medium or high but in general an approximately atmospheric or slightly superatmospheric pressure of hydrogen is preferred. The transition metal catalyst employed is preferably palladium, for example palladium on charcoal, palladium on barium sulphate or palladium on calcium carbonate. The hydrogenation may be effected in any convenient solvent in which the ester is soluble such as tetrahydrofuran, ethyl acetate, ethanol or aqueous ethanol. If this hydrogenation is carried out in the presence of a base then a salt of the compound of formula (I) is produced. Suitable bases for inclusion include $NaHCO_3$, $Na_2CO_3$, $K_2CO_3$, $CaCO_3$, $MgCO_3$ and $LiHCO_3$. If no base is present then hydrogenation leads to the preparation of an acid of formula (I) which may then be neutralised if desired to yield a salt. Suitable bases for such neutralisa-

tion include LiOH, $NaHCO_3$, KOH, $Ca(OH)_2$, $Ba(OH)_2$, $Mg(OH)_2$, $NH_4OH$, $N(C_2H_5)_3$, Na ethylhexanoate, K ethylhexanoate and the like e.g. MgO and $NH_2C(CH_3)_3$.

The lithium salts of the compounds of formula (I) tend to be more easily prepared in pure crystalline form than other salts of the compounds of formula (I). It is therefore often convenient to first form the lithium salt and then convert this into a further salt by ion-exchange, for example by passing a solution of the lithium salt through a bed of cation exchange resin in sodium, potassium, calcium or ammonium form. Suitable cation exchange resins include Amberlite IR 120® and equivalent resins. Another salt suitable for use as an intermediate in this way is the t-butylamine salt.

Oxidation of a compound of the formula (I) or a salt or cleavable ester thereof wherein X is a sulphur atom may be effected by reaction with an organic per acid such as peracetic acid, meta-periodic acid and m-chloroperbenzoic acid. Normally one equivalent of the acid is used for oxidation to the sulfoxide level whereas two or more equivalents are used for oxidation to the sulfone level. The reaction is generally carried out at a depressed temperature which may conveniently be at about 0°. Suitable solvents are inert organic solvents such as dichloromethane and chloroform.

In a further aspect of this invention there is provided a process for the preparation of a compound of the formula (I) as hereinbefore defined or a salt or cleavable ester thereof which process comprises the reaction of a cleavable ester of clavulanic acid with a compound of the formula (III):

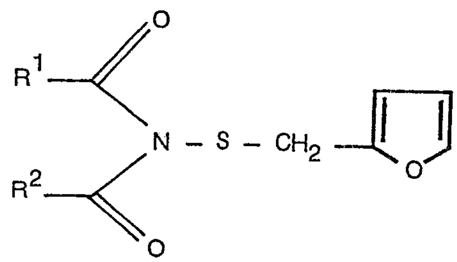

(III)

and a compound of the formula (IV):

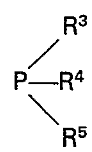

(IV)

in which $R^1$ and $R^2$ are moieties such that the compound of the formula (III) is a sulphenimide; $R^3$, $R^4$ and $R^5$ are groups such that the compound of the formula (IV) is a phosphine; and thereafter if desired:

a) oxidising the sulphur atoms to a SO or $SO_2$ group,

b) cleaving the cleavable ester to yield the compound of the formula (I) or its salt,

c) re-esterifying the compound of the formula (I) or its salt to form a different cleavable ester.

The groups $R^1$ and $R^2$ will normally be selected from hydrocarbon groups which may be linked together. Particularly apt groups $R^1$ and $R^2$ include lower alkyl groups optionally linked to form part of a 5- or 6-membered ring.

Preferred compounds of the formula (II) include those of the formulae (V) and (VI):

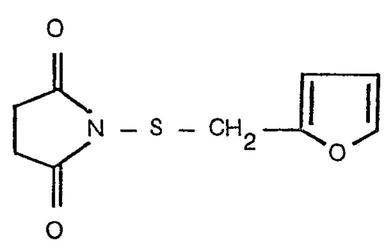

(V)

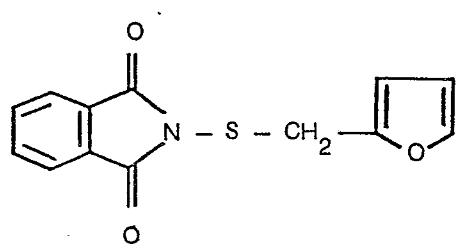

(VI)

The compound of the formula (V) is particularly suitable as a by-product of the reaction is water-soluble and may thus be readily removed by washing with water.

Suitable values for $R^3$, $R^4$ and $R^5$ include hydrocarbon groups of up to 7 carbon atoms and hydrocarbon groups substituted by a group of the sub-formulae $OR^6$ or $O.CO.R^6$ where $R^6$ is a lower alkyl group.

Most suitably $R^3$, $R^4$ and $R^5$ each have the same meaning.

Favoured values for $R^3$, $R^4$ and $R^5$ include methyl, ethyl, n-propyl, n-butyl, phenyl, 4-methoxyphenyl and benzyl.

A particularly suitable compound of the formula is tri-n-butyl-phosphine.

The reaction of a cleavable ester of clavulanic acid and the compounds of the formulae (III) and (IV), may take place at any convenient non-extreme temperature, for example −10°C to 50°C, more usually 0° to 40°C, generally 10° to 30° and very conveniently at ambient (15—25°C). The reaction is generally carried out in an aprotic medium. Suitable solvents include hydrocarbon solvents such as benzene and toluene.

If desired the reaction may be carried out under an inert gas such as nitrogen.

Once the reaction is complete, for example as indicated by tlc, the mixture may be washed with water to remove the water-soluble products, and the organic layer dried and evaporated. The initial product may be further purified by chromatography if desired, for example over silica eluting with ethyl acetate/petroleum ether.

Once formed the ester of the thioether may optionally be oxidised and/or converted to the corresponding acid or salt or to another ester in conventional manner.

The following Examples illustrate the invention.

Example 1

*p-Nitrobenzyl 9-(2'-furylmethylthio)-9-deoxyclavulanate*

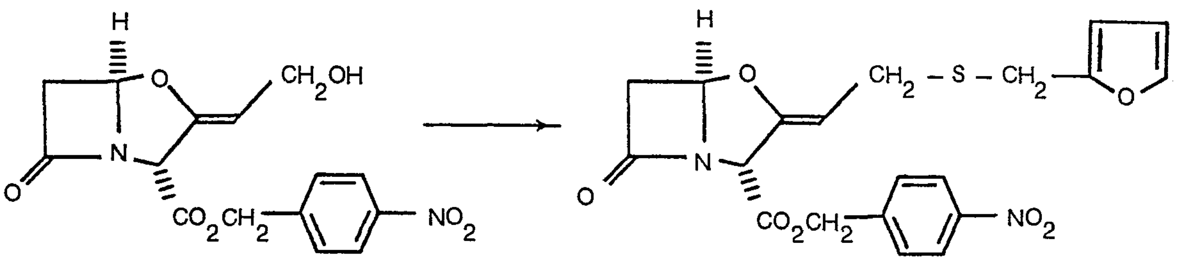

(e1) (e2)

p-Nitrobenzyl clavulanate (el) (2 g) was dissolved in methylene dichloride (100 ml) and cooled to −30°C under $N_2$. Boron trifluoride-etherate (0.36 ml) was added at −30° followed by a solution of furfurylmercaptan (0.62 ml) in methylene chloride (10 ml). The mixture was stirred under nitrogen at −10° for 2 hr. and washed with 3% sodium bicarbonate solution (2 × 100 ml) and water (50 ml). The organic phase was dried over anhydrous magnesium sulphate and the solvent evaporated to give an oil which after chromatography on silica-gel using ethyl acetate — petrol 1:3 as eluent gave p-nitrobenzyl-9-(2'-furylmethylthio)-9-deoxyclavulanate (e2) as an oil (449 mg). $[\alpha]_D^{20}$ 21.1° (c. 1.8; $CHCl_3$); $\upsilon_{max}$ ($CHCl_3$) 1805, 1755 and 1692 $cm^{-1}$; $\lambda_{max}$ (EtOH) 217 (22,500) and 267.5 (11,150) nm: $\delta$ ($CDCl_3$) 3.03 (1H, d, $J$ 17 Hz, 6$\beta$—C$H$), 3.22 (2H, d $J$ 8 Hz, 9—C$H_2$), 3.49 (1H, dd, $J$ 17 and 2.5 Hz, 6$\alpha$—C$H$), 3.60 (2H, s, —SC$H_2$—Ar), 4.70 (1H, br.t, $J$ Hz, 8—C$H$), 5.10 (1H, s, 3—C$H$), 5.26 (2H, s —OC$H_2$Ar), 5.65 (1H, d, $J$ 2.5 Hz, 5—C$H$), 6.0—6.4 (2H, m furyl 3$H$ and 4$H$), 7.2—7.4 (1H, m, furyl 5—C$H$), 7.49 and 8.20 (4H, 2d, Ar—$H$).

Example 2

*Lithium 9-(2'-furylmethylthio)-9-deoxyclavulanate*

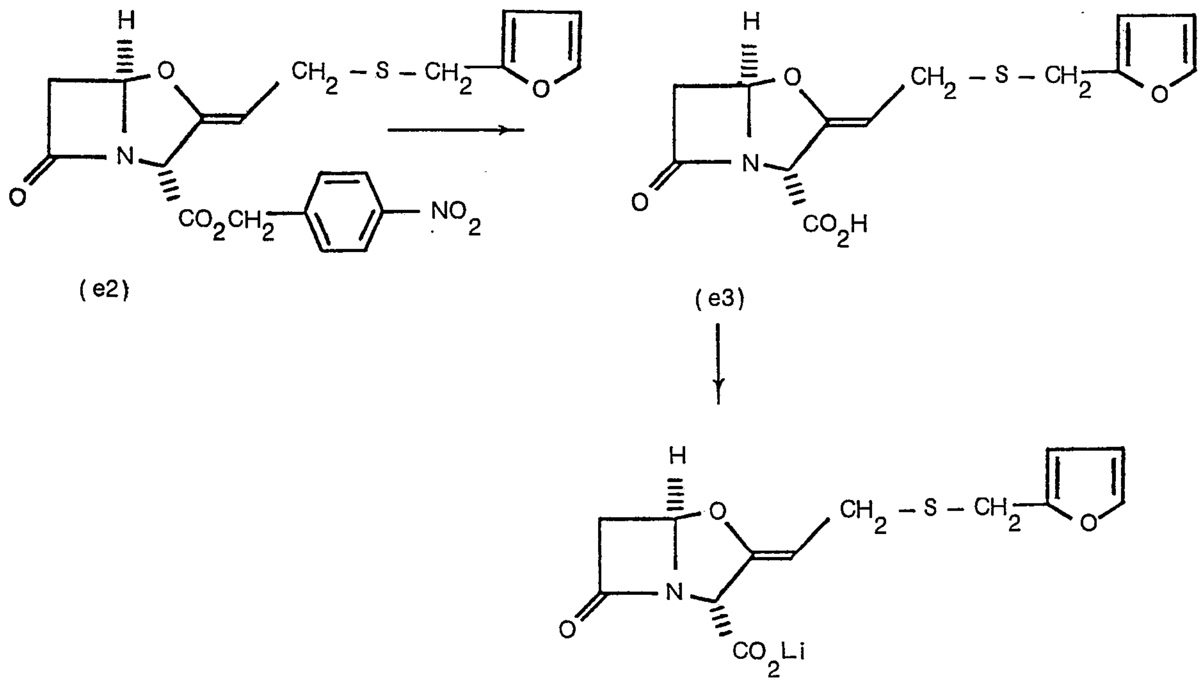

(e2) (e3) (e4)

A solution of p-nitrobenzyl 9-(2′-furylmethylthio)-9-deoxyclavulanate (e2) (.35 g, .81 mmole) in tetrahydrofuran (50 ml) was added to a pre-hydrogenated suspension of 10% Pd/C in tetrahydrofuran (50 ml). The mixture was hydrogenated at 1 atmosphere for $1\frac{1}{2}$ hours, filtered through celite and the filtrate evaporated to 10 ml. At this point the solution contained 9-(2′-furylmethylthio)-9-deoxyclavulanic acid (e3). A solution of lithium carbonate (.03 g, .405 mmole) in water (20 ml) was added with stirring and the resulting solution extracted with ethyl acetate (3 x 20 ml), acidified to pH 2.5 with 1N hydrochloric acid and extracted with ethyl acetate (3 x 20 ml). The latter ethyl acetate extracts were dried over $MgSO_4$ and evaporated. The residue was taken up in 10 ml tetrahydrofuran, 10 ml water added and treated with .1N aqueous lithium carbonate to bring the pH to 7. The organic solvent was evaporated and the remaining aqueous solution freeze dried to provide lithium 9-(2′-furylmethylthio)-9-deoxyclavulanate (e4) (.122 g, 50%). $[\alpha]_D^{20}$ + 60.2° (c. 1.0; $H_2O$); $\upsilon_{max}$ (KBr) 1765, 1690 and 1615 $cm^{-1}$; $\lambda_{max}$ ($H_2O$) 201 nm (15150) and 222 nm (14850); $\delta$ ($D_2O$ — HOD at 4.61) 3.00 (1H, d, *J* 17 Hz, 6$\beta$—C*H*), 3.20 (2H, d, *J* 8 Hz, 9—C*H*$_2$), 3.52 (1H, dd, *J* 17 and 2.5 Hz, 6$\alpha$—C*H*), 3.71 (2H, s, —SC*H*$_2$Ar), 4.72 (1H, broad t, *J* 8 Hz, 8—C*H*), 4.87 (1H, s, 3—C*H*), 5.64 (1H, d, *J* 2.5 Hz, 5—C*H*), 6.2—6.5 (2H, m, furan 3—H and 4—H), 7.4—7.5 (1H, m, furan 5—H).

Example 3

*p-Nitrobenzyl 9-(2'-furylmethylsulfonyl)-9-deoxyclavulanate*

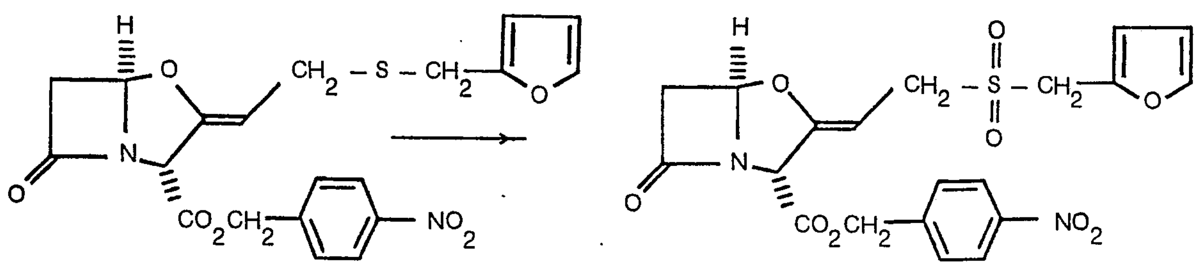

(e2) (e5)

A solution of p-nitrobenzyl 9-(2′-furylmethylthio)-9-deoxyclavulanate (e2) (1.20 g, 2.78 mmole) in 50 ml dichloromethane was ice cooled and treated dropwise with a solution of m-chloroperbenzoic acid (1.33 g, 7.73 mmol) in dichloromethane (20 ml). The mixture was stirred 2 hour, washed with

saturated aqueous sodium bicarbonate (2 x 50 ml) and water (50 ml), dried over $MgSO_4$ and evaporated. The residue was chromatographed on silica, eluting with ethyl acetate/petrol 4:6, to yield p-nitrobenzyl 9-(2′-furylmethylsulfonyl)-9-deoxyclavulanate (e5) (.95 g, 73%) as a white foam. $[\alpha]_D^{20}$ + 10.3° (c. 1.1; $CHCl_3$); $\upsilon_{max}$ ($CHCl_3$) 1810, 1760 and 1695 $cm^{-1}$; $\lambda_{max}$ (EtOH) 224 nm (s) (23400) and 263 nm (13280); $\delta$ ($CDCl_3$) 3.09 (1H, d, *J* 17 Hz, 6β—C*H*), 3.3—3.9 (3H, m, 6α—C*H* and 9—C$H_2$), 4.22 (2H,S—SO,$CH_2$), 4.78 (1H, t, *J* 8 Hz, 8—C*H*), 5.18 (1H, s, 3—C*H*), 5.27 (2H, s, —OC$H_2$Ar), 5.74 (1H, d, *J* 2.5 Hz, 5—C*H*), 6.2—6.5 (2H, m, furyl 3—H and 4—H), 7.2—7.6 (3H, m, furyl 5—H and 2 Ar-H of p-nitrobenzyl,), 8.19 (2H, d, 2 Ar-H of p-nitrobenzyl).

## Example 4

*Lithium 9-(2′-furylmethylsulfonyl)-9-deoxyclavulanate*

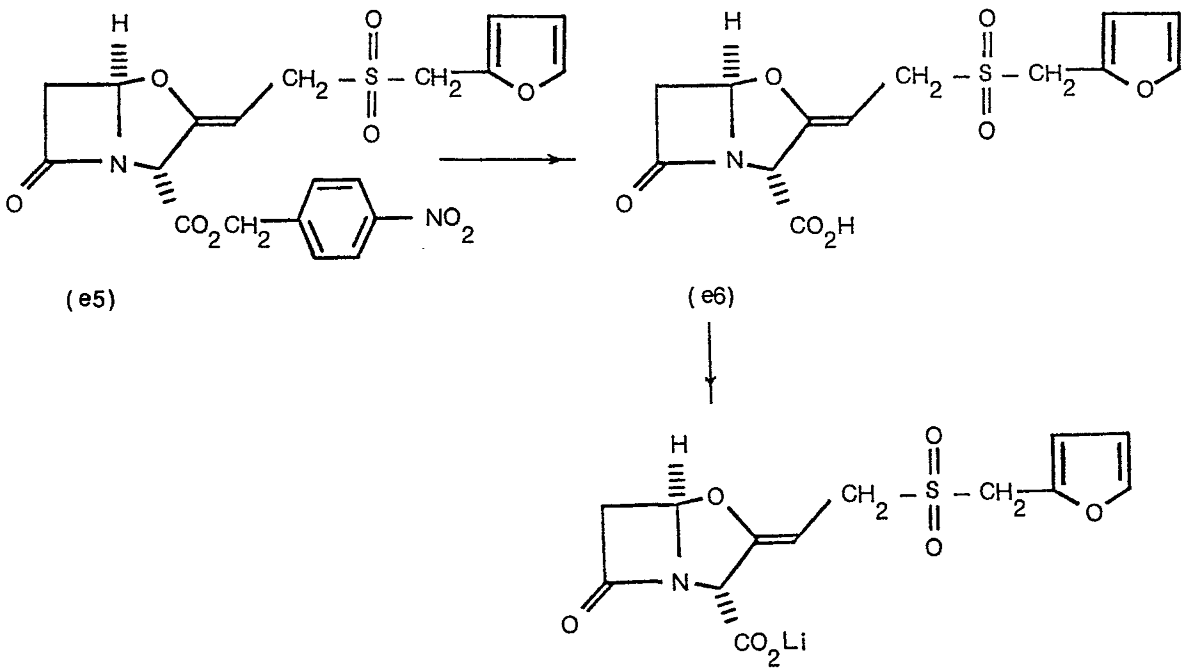

(e5) (e6)

(e7)

A solution of p-nitrobenzyl 9-(2′-furylmethylsulfonyl)-9-deoxyclavulanate (e5) (.95 g, 2.04 mmole) in tetrahydrofuran (100 ml) was added to a prehydrogenated suspension of 10% Pd/C (1 g) in tetrahydrofuran (20 ml). The mixture was hydrogenated at 1 atmosphere for 20 mins, filtered through celite and the filtrate evaporated to 10 ml to yield a solution of 9-(2′-furylmethylsulfonyl)-9-deoxyclavulanic acid (e6). A solution of lithium carbonate (0.075 g, 1.02 mmole) in water (100 ml) was added and the solution extracted with ethyl acetate (3 x 50 ml), adjusted to pH 7 with 1N hydrochloric acid and freeze dried to yield lithium 9-(2′-furylmethylsulfonyl)-9-deoxyclavulanate (e7) (.60 g, 87%) as a white solid. $[\alpha]_D^{20}$ + 33.7° (c. 1.0; $H_2O$); $\upsilon_{max}$ (KBr) 1780, 1690 and 1620 $cm^{-1}$. $\lambda_{max}$ ($H_2O$) 206 nm (s) (13740) and 224 nm (15080); $\delta$ ($D_2O$ — HOD at 4.61) 3.10 (1H, d, *J* 17 Hz, 6β—C*H*), 3.59 (1H, dd, *J* 17 and 2.5 Hz, 6α—C*H*), 4.01 (2H, d, *J* 8 Hz, 9—C$H_2$), 5.00 (1H, s, 3—C*H*), 5.76 (1H, d, *J* 2.5 Hz, 5—C*H*), 6.3—6.7 (2H, m, furyl 3—H and 4—H), 7.4—7.6 (1H, m, furyl 5—H). 8—C*H* and —$SO_2CH_2$ obscured by HOD.

## Example 5

*p-Nitrobenzyl 9-(2′-furylmethylsulfinyl)-9-deoxyclavulanate*

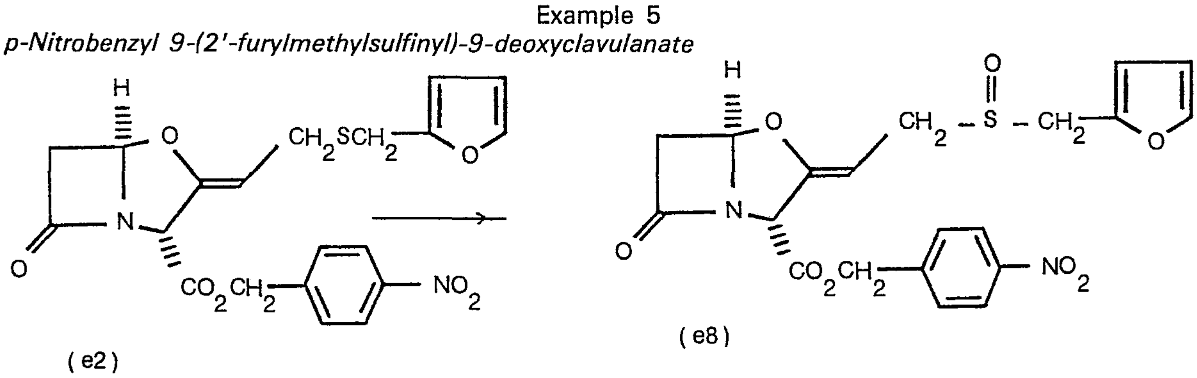

(e2) (e8)

A stirred, ice cooled solution of p-nitrobenzyl 9-(2′-furylmethylthio)-9-deoxyclavulanate (e2) (.375 g, .87 mmole) in dichloromethane (25 ml) was treated over 5 mins dropwise with a solution of m-chloroperbenzoic acid (.150 g, .87 mmole) in dichloromethane (10 ml). The solution was stirred 1½ hrs. washed with aqueous 1N sodium bicarbonate solution (2 × 40 ml) and water (40 ml), dried over $MgSO_4$ and evaporated. The residue was chromatographed on silica (30 g), eluting with ethyl acetate to provide p-nitrobenzyl-(2′-furylmethylsulfinyl)-9-deoxyclavulanate (e8) (.29 g, 74%) as a colourless oil. $[\alpha]_D^{20}$ + 6.0° (c. 1.0; $CHCl_3$); $\nu_{max}$ ($CHCl_3$) 1810, 1755 and 1690 $cm^{-1}$; $\lambda_{max}$ (EtOH) 230 nm (s) (19270) and 260 nm (s) (10040); $\delta$ ($CDCl_3$) 3.09 (1H, d, $J$ 17 Hz, 6$\beta$—C$H$), 3.3—3.7 (3H, m, 6$\alpha$—C$H$ and 9—C$H_2$), 3.92 and 3.97 (2H, 2s-C$H_2$-furyl), 4.84 (1H, broad t, $J$ 8 Hz, 8—C$H$), 5.20 (1H, s, 3—C$H$), 5.28 (2H, s, —OC$H_2$Ar), 5.72 (1H, d, $J$ 2.5 Hz, 5—C$H$) 6.3—6.5 (2H, m, furyl 3—H and 4—H), 7.3—7.6 (3H, m, furyl 5—H and 2 p-nitrophenyl-H), 8.20 (2H, d, 2 p-nitrophenyl-H).

Example 6

*Lithium 9-(2′-furylmethylsulfinyl)-9-deoxyclavulanate*

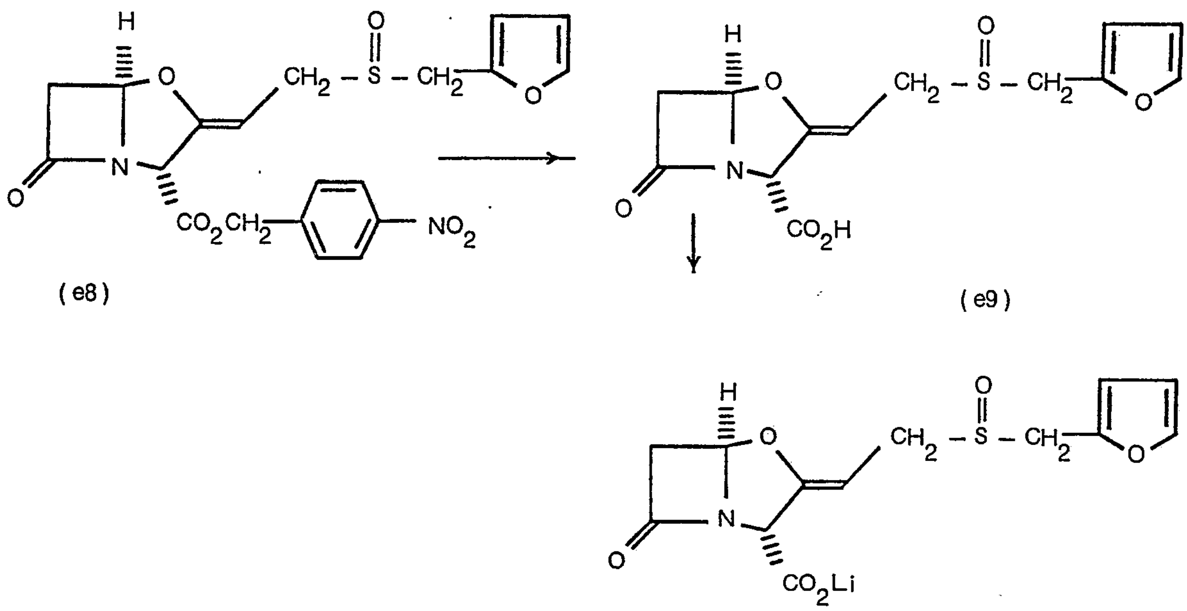

(e8)

(e9)

(e10)

A solution of p-nitrobenzyl 9-(2′-furylmethylsulfinyl)-9-deoxyclavulanate (.250 g .56 mmole) in tetrahydrofuran (50 ml) was added to a pre-hydrogenated suspension of 10% Pd/C (.40 g) in tetrahydrofuran (50 ml) and hydrogenation at 1 atmosphere continued for 1½ hrs. The mixture was filtered through celite and the filtrate evaporated to 10 ml to yield a solution of 9-(2′-furylmethylsulfinyl)-9-deoxyclavulanic acid (e9). Water (10 ml) was added, followed by .1M aqueous lithium carbonate solution (2.8 ml), and the solution was washed with ethyl acetate (3 × 20 ml), saturated with sodium chloride, acidified to pH 2.5 with 1N hydrochloric acid and extracted with ethyl acetate (3 × 20 ml). The combined extracts were dried over $MgSO_4$ and evaporated and the residue taken up in tetrahydrofuran (10 ml). Water (10 ml) was added and the solution brought to pH 7 with .1M aqueous lithium carbonate solution. Organic solvent was removed by evaporation water reduced pressure and the aqueous solution freeze dried to yield lithium 9-(2′-furylmethylsulfinyl)-9-deoxyclavulanate (e10) (.133 g, 75%) as a yellow solid. $[\alpha]_D^{20}$ + 23.2° (c. 1.2; $H_2O$), $\nu_{max}$ (KBr) 1785, 1685 and 1620 $cm^{-1}$; $\lambda_{max}$ ($H_2O$) 205 nm (s) (14670) and 231 nm (18100); $\delta$ ($D_2O$ — HOD at 4.61) 3.07 1H, d $J$ 17 Hz, 6$\beta$—C$H$), 3.3—3.8 (3H, m, 6$\alpha$—C$H$ and 9—C$H_2$), 4.05 and 4.30 (2H, 2 parts of ABq, $JAB$ 14.5 Hz, —C$H_2$-furyl), 4.77 (1H, t, $J$ 8 Hz, 8—C$H$), 5.00 (1H, s, 3—C$H$), 5.73 (1H, d, $J$ 2.5 Hz, 5—C$H$), 6.49 (2H, broad s, furyl 3H and 4H), 7.55 (1H, broad s, furyl 5—H).

Example 7

*Sodium 9-(2′-furylmethylsulfonyl)-9-deoxyclavulanate*

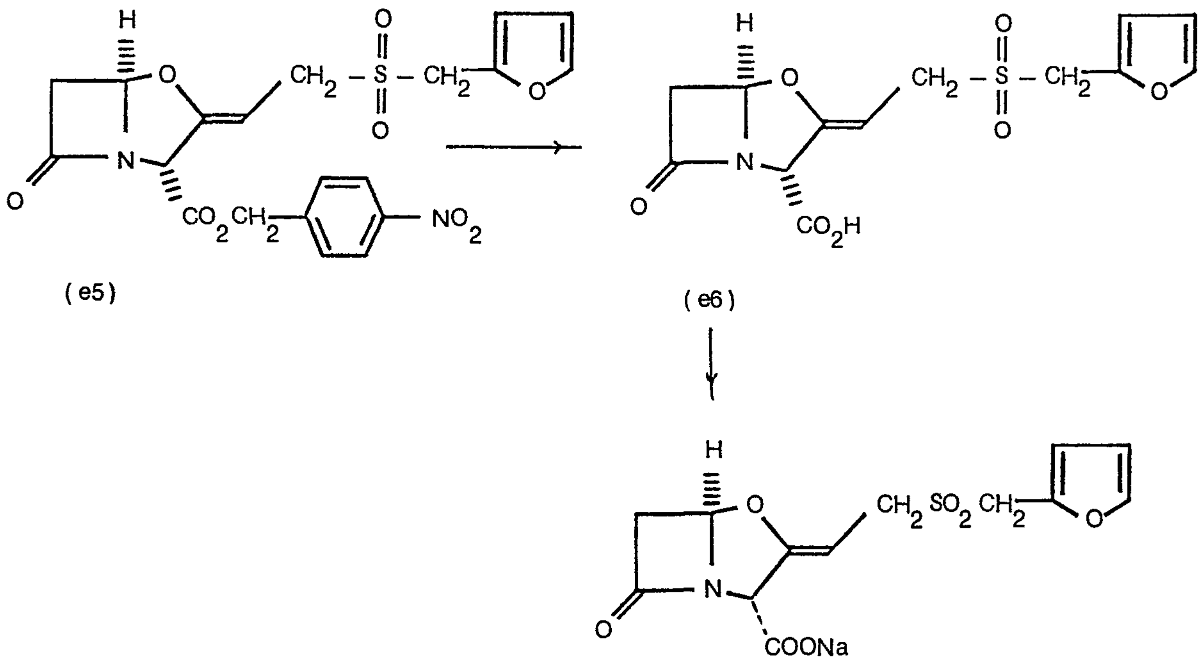

(e5) (e6) (e11)

A solution of p-nitrobenzyl 9-(2′-furylmethylsulfonyl)-9-deoxyclavulanate (1.14 g 2.46 mmole) in tetrahydrofuran (50 ml) was added to a pre-hydrogenated suspension of 10% Pd/C (1.2 g) in tetrahydrofuran (50 ml) and hydrogenation continued for 30 mins at 1 atmosphere. The mixture was filtered through celite and the filtrate evaporated under reduced pressure to approx. 10 ml, ice cooled and stirred, and treated with a solution of sodium bicarbonate (.206 g 2.46 mmole) in 50 ml water. The resulting mixture was washed with ethyl acetate (3 x 50 ml), adjusted to pH 7 with .1N hydrochloric acid and freeze dried to provide sodium 9-(2′-furylmethylsulfonyl)-9-deoxyclavulanate (e11) (.73 g, 85%) as a pale yellow solid. $[\alpha]_D^{20}$ + 30.4° (c.1.1;$H_2O$); $\upsilon_{max}$ (KBr) 1785, 1690 $cm^{-1}$; $\lambda_{max}$ ($H_2O$) 210 nm (s) (12020) and 227 nm (13640); $\delta$ ($D_2O$—MeCN=2.00) 3.06 (1H, d, *J* 17.5 Hz, 6$\beta$—C*H*), 3.53 (1H, dd, *J* 17.5 and 2.5 Hz, 6$\alpha$—C*H*), 3.97 (2H, d, *J* 8 Hz, 8—C$H_2$), 4.98 (1H, s, 3—C*H*), 5.73 1H, d, *J* 2.5 Hz, 5—C*H*), 6.4—6.6 (2H, m furyl 3—H and 4—H), 7.5—.6 (1H, m, furyl 5—H).

Example 8

*p-Nitrobenzyl 9-(2′-furylmethylthio)-9-deoxyclavulanate*

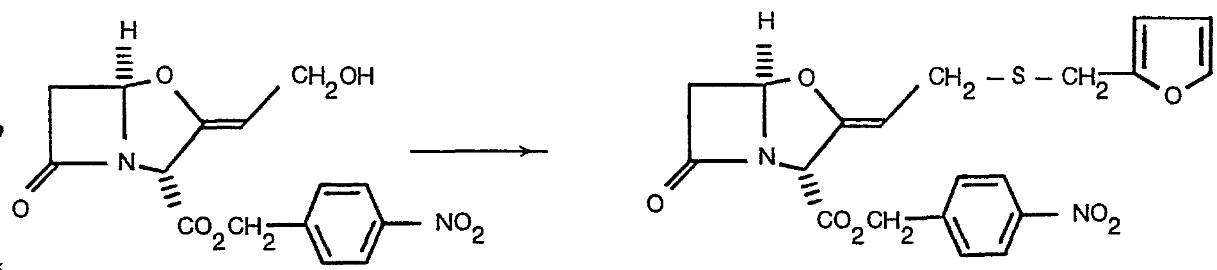

(e1) (e2)

Tri-n-butyl phosphine (.215 ml .87 mmole) and p-nitrobenzyl clavulanate (e1) (.167 g .50 mmole) were dissolved in tetrahydrofuran (5 ml) under nitrogen and treated with N-furfurylthio succinimide (.184 g .87 mmole) portion wise over 20 mins. The solution was diluted with ethyl acetate (20 ml), washed with water (2 x 20 ml), dried over $MgSO_4$ and evaporated. Chromatography of the residue on silica (15 g) eluting with ethyl acetate/petrol 1:4 provided p-nitrobenzyl 9-(2′-furylmethylthio)-9-deoxy clavulanate (e2) (0.056 g, 26%) as an oil.

### Example 9

*p-Nitrobenzyl-9-(2'-furylmethylthio)-9-deoxyclavulanate*

A solution of p-nitrobenzyl-9-0-dichloroacetyl clavulanate (0.446 g) and furfurylmercaptan (0.2 ml) in dimethylformamide (10 ml) was cooled to −50°C, triethylamine (0.14 ml) in dimethylformamide (5 ml) was added dropwise with stirring and the reaction mixture was allowed to warm to 0°C. The mixture was diluted with ethyl acetate (100 ml), washed with water (100 ml), dilute hydrochloric acid (50 ml), water (100 ml), dried over anhydrous magnesium sulphate and evaporated in vacuo. The residue was chromatographed on silica using ethyl acetate/petrol 1:4 as eluant to provide the title ester as an oil (0.22 g; 53%), identical in all respects to Example 1.

### Example 10

*Composition*

a. Amoxycillin trihydrate (equivalent to 250 mg of amoxycillin) sodium 9-(2'-furylmethylsulfonyl)-9-deoxyclavulanate (equivalent to 125 mg of 9-(2'-furylmethylsulfonyl)-9-deoxyclavulanic acid) are filled into a two part gelatin capsule.

b. Sodium amoxycillin (equivalent to 250 mg of amoxycillin) and sodium 9-(2'-furylmethylsulfonyl)-9-deoxyclavulanate (equivalent to 125 mg of 9-(2'-furylmethyl-sulfonyl)-9-deoxyclavulanic acid) were dissolved in 2 ml of sterile water for injection B.P. to yield a solution suitable for injection.

Compositions such as those described above and other synergistic compositions of this invention may be used to treat infections due to strains of Staphyloccocus aureus, Klebsiella aerogenes, E. coli, Proteus spp., Bacteriodes spp., Haemophilus influenza and Neisseria gonorrhoeae.

### Description 1

*N-Furylthiosuccinimide*

A solution of furfuryl mercaptan (5.7 g) in benzene (100 ml) was treated with 50 g Merck basic aluminium oxide (70—230 mesh) and stirred 24 hrs. with air bubbling through. The suspension was filtered and the filtrate evaporated to leave furfuryl disulfide as a reddish oil.

A solution of furfuryl disulfide (3.43 g .0152 mole) in carbon tetrachloride (10 ml) was ice cooled and treated dropwise with a solution of chlorine (1.08 g .0152 mole) in carbon tetrachloride (15 ml). The resulting solution was added dropwise over 15 mins to a solution of succinimide (3.01 g .0304 mole) and triethylamine (4.58 ml .033 mole) in dimethylformamide (30 ml), and the mixture stirred 1 hour, diluted with ethyl acetate (300 ml), washed with water (2 × 100 ml), dried over $MgSO_4$ and evaporated. The residue was chromatographed on silica (50 g) eluting with ethyl acetate/petrol (1:1), and the required compound crystallised from ethyl acetate (.65 g), Prisms, m.p. 124—7°C, $\nu_{max}$ ($CHCl_3$) 1730 cm$^{-1}$; $\delta$ ($CDCl_3$) 2.73 (4H, s, —$CH_2CH_2$—), 4.08 (2H, s, —$SCH_2$—), 6.1—6.4 (2H, m, furyl 3—H and 4—H), 7.3—7.5 (1H, m, furyl 5—H); Found: C, 51.2; H, 4.2; N, 6.6, S, 15.2% $C_9H_9NO_3S$ requires C, 51.2, H, 4.3, N, 6.6, S, 15.2%.

### Description 2

*Pharmacology*

The compound of Example 7 was found to reduce the $CD_{50}$ of amoxycillin from >1000 mg/kg to 6.4 mg/kg in mice infected with intra peritoneally with a plasmid carrying $\beta$-lactamase producing strain of E. coli with oral doseing at 1,3 and 5 hour past infection using 20 mg/kg of said compound of Example 7. Under similar conditions the analogous compound containing a phenyl ring in place of the furyl ring only reduced the $CD_{50}$ of amoxycillin to about 100 mg/kg.

When administered sub-cutaneously at 1 and 5 hours past infection the compound of Examples 4 and 6 reduced the $CD_{50}$ values of amoxycillin to 6.6 and 15 mg/kg respectively in the above infection using 2 mg/kg of said compound of Examples 4 and 6.

No drug induced toxic effects were noted in the test animals at the therapeutic dose.

## Claims

1. A compound of the formula (I):

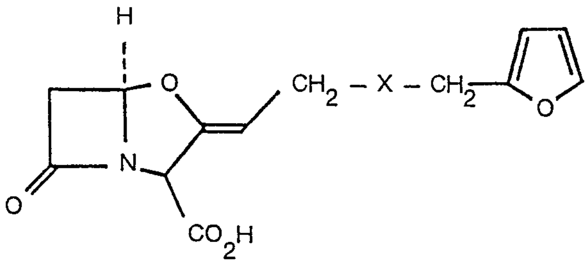

(I)

or a salt or cleavable ester thereof wherein X is a sulphur atom or a SO or $SO_2$ group.

2. A compound as claimed in claim 1 in the form of a pharmaceutically acceptable salt or pharmaceutically acceptable ester.

3. A compound as claimed in either claim 1 or claim 2 wherein X is a $SO_2$ group.

4. A compound as claimed in either claim 2 or 3 in the form of a sodium, potassium calcium or magnesium salt.

5. A compound as claimed in either claims 2 or 3 in the form of an acetoxymethyl, acetoxyethyl, phthalidyl, ethoxycarbonyloxymethyl, $\alpha$-ethoxycarbonyloxyethyl or pivaloyloxymethyl ester.

6. A pharmaceutical composition which comprises a compound as claimed in claim 2 and a pharmaceutically acceptable carrier therefor.

7. A pharmaceutical composition as claimed in claim 6 which also comprises a penicillin or cephalosporin.

8. A process for the preparation of a compound as claimed in claim 1 which process comprises the reaction of a cleavable ester of a compound of the formula (II):

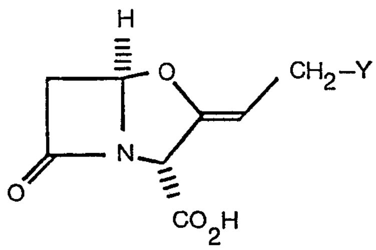

(II)

wherein Y is a displaceable atom or group; with the furfurylmercaptan to yield the cleavable ester of the compound of the formula (I) wherein X is a sulphur atom, and thereafter optionally performing one or more the following steps:

a) oxidising the sulphur atom to a SO or $SO_2$ group.

b) cleaving the cleavable ester to yield the compound of the formula (I) or its salt,

c) re-esterifying the compound of the formula (I) or its salt to form a different cleavable ester.

9. A process for the preparation of a compound as claimed in claim 1 which process comprises the reaction of a cleavable ester of clavulanic acid with a compound of the formula (III):

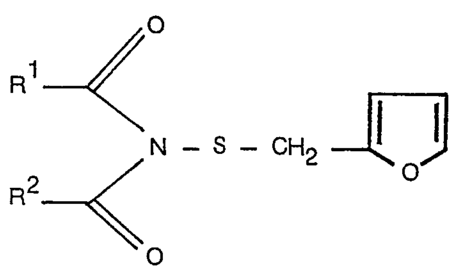

(III)

and a compound of the formula (IV):

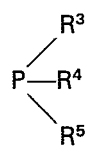

(IV)

in which $R^1$ and $R^2$ are moieties such that the compound of the formula (III) is a sulphenimide; $R^3$, $R^4$ and $R^5$ are groups such that the compound of the formula (IV) is a phosphine; and thereafter if desired.

a) oxidising the sulphur atom to a SO or $SO_2$ group.

b) cleaving the cleavable ester to yield the compound of the formula (I) or its salt,

c) re-esterifying the compound of the formula (I) or its salt to form a different cleavable ester.

**Revendications**

1. Composé de formule (I):

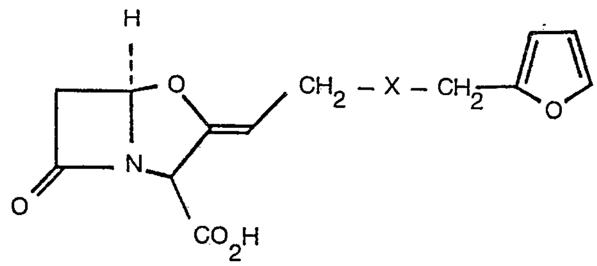

(I)

ou sel ou ester dissociable de celui-ci, formule dans laquelle X est un atome de soufre ou un groupe SO ou $SO_2$.

2. Composé suivant la revendication 1, sous la forme d'un sel pharmaceutiquement acceptable ou d'un ester pharmaceutiquement acceptable.

3. Composé suivant la revendication 1 ou 2, selon lequel X est un groupe $SO_2$.

4. Composé suivant la revendication 2 ou 3, sous la forme d'un sel de sodium, de potassium, de calcium ou de magnésium.

5. Composé suivant la revendication 2 ou 3, sous la forme d'un ester acétoxyméthylique, acétoxy-éthylique, phtalidylique, éthoxycarbonyloxyméthylique, $\alpha$-éthoxycarbonyloxyéthylique ou pivaloyloxy-méthilique.

6. Composition pharmaceutique renfermant un composé suivant la revendication 2 et un véhicule ou excipient pharmaceutiquement acceptable pour ce composé.

7. Composition pharmaceutique suivant la revendication 6, renfermant également un pénicilline ou une céphalosporine.

8. Procédé pour la préparation d'un composé suivant la revendication 1, caractérisé en ce qu'on fait réagir un ester dissociable d'un composé de formule (II):

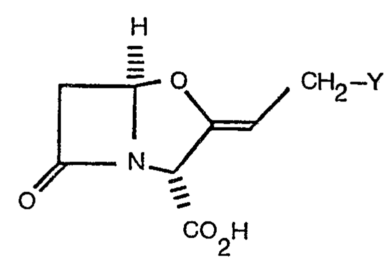

(II)

dans laquelle Y est un atome ou groupe déplaçable ou séparable avec du furfuryl-mercaptan, pour donner l'ester dissociable du composé de formule (I) dans laquelle X est un atome de soufre puis, éventuellement, on effectue un ou plusieurs des stades opératoires ci-après:

a) oxydation de l'atome de soufre en un groupe SO ou $SO_2$.

b) dissociation de l'ester dissociable pour donner le composé de formule (I) ou son sel;

c) ré-estérification du composé de formule (I) ou de son sel pour former un ester dissociable différent.

9. Procédé pour la préparation d'un composé suivant la revendication 1, caractérisé en ce qu'on effectue la réaction d'un ester dissociable d'acide clavulanique avec un composé de formule (III):

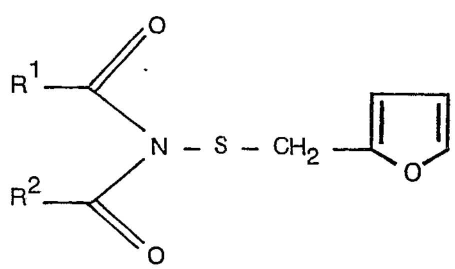

(III)

et un composé de formule (IV):

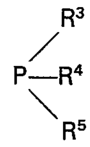

(IV)

formules dans lesquelles $R^1$ et $R^2$ sont des fractions molaires telles que le composé de formule (III) soit un sulfénimide; $R^3$, $R^4$ et $R^5$ sont des groupes tels que le composé de formule (IV) soit une phosphine, puis si désiré:

a) on oxyde l'atome de soufre en un groupe SO ou $SO_2$;

b) on dissocie l'ester dissociable pour donner le composé de formule (I) ou son sel;

c) on ré-estérifie le composé de formule (I) ou son sel pour former un ester dissociable différent.

**Patentansprüche**

1. Eine Verbindung der Formel (I):

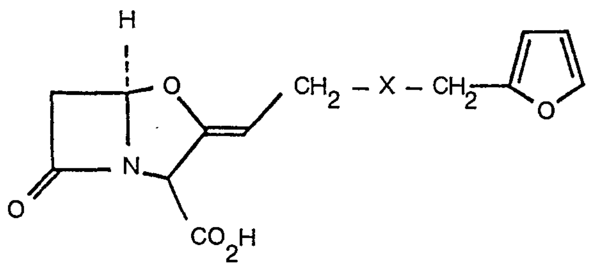

(I)

oder ein Salz oder spaltbarer Ester derselben, wobei X ein Schwefelatom oder eine SO- oder $SO_2$-Gruppe ist.

2. Eine Verbindung, wie in Anspruch 1 beansprucht, in Form eines pharmakologisch verträglichen Salzes oder pharmakologisch verträglichen Esters.

3. Eine Verbindung, wie entweder in Anspruch 1 oder Anspruch 2 in beansprucht, in der X eine $SO_2$-Gruppe ist.

4. Eine Verbindung, wie entweder in Anspruch 2 oder 3 beansprucht, in Form eines Natrium-, Kalium-, Calcium- oder Magnesiumsalzes.

5. Eine Verbindung, wie entweder in Anspruch 2 oder 3 beansprucht, in Form eines Acetoxymethyl-, Acetoxyäthyl-, Phthalidyl-, Äthoxycarbonyloxymethyl-, $\alpha$-Äthoxycarbonyloxyäthyl- oder Pivaloyloxymethylesters.

6. Eine pharmazeutische Zusammensetzung, die eine Verbindung wie in Anspruch 2 beansprucht und einen pharmakologisch verträglichen Träger dafür enthält.

7. Eine pharmazeutische Zusammensetzung, wie in Anspruch 6 beansprucht, die auch ein Penicillin oder Cephalosporin enthält.

8. Ein Verfahren zur Herstellung einer Verbindung, wie in Anspruch 1 beansprucht, die Maßnahmen umfassend, daß ein spaltbarer Ester eine Verbindung der Formel (II):

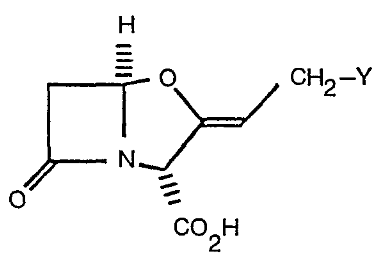

(II)

in der Y ein(e) verdrängbare(s) Atom oder Gruppe ist, mit dem Furfurylmercaptan zu dem spaltbaren Ester der Verbindung der Formel (I) umgesetzt wird, in der X ein Schwefelatom ist, und anschließend gegebenenfalls einer oder mehrere der folgenden Schritte durchgeführt werden:

a) Oxidieren des Schwefelatoms zu einer SO- oder $SO_2$-Gruppe,

b) Spalten des spaltbaren Esters zur Verbindung der Formel (I) oder deren Salz,

c) Wiederverestern der Verbindung der Formel (I) oder deren Salz zu einem anderen spaltbaren Ester.

9. Ein Verfahren zur Herstellung einer Verbindung, wie in Anspruch 1 beansprucht, die Maßnahmen umfassend, daß ein spaltbarer Ester der Clavulansäure mit einer Verbindung der Formel (III):

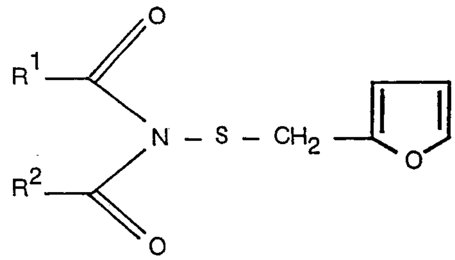

(III)

und einer Verbindung der Formel (IV) umgesetzt wird:

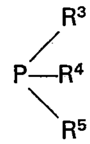

(IV)

in der $R^1$ und $R^2$ solche Reste sind, daß die Verbindung der Formel (III) ein Sulphenimid ist, $R^3$, $R^4$ und $R^5$ solche Gruppen sind, daß die Verbindung der Formel (IV) ein Phosphin ist, und anschließend, wenn es erwünscht ist,

a) das Schwefelatom zu einer SO- oder $SO_2$-Gruppe oxidiert wird,

b) der spaltbare Ester in die Verbindung der Formel (I) oder deren Salz gespalten wird,

c) die Verbindung der Formel (I) oder deren Salz zu einem anderen spaltbaren Ester wiederverestert wird.